# EUROPEAN PATENT SPECIFICATION

(11) **EP 1 139 865 B1**
(45) Date of publication and mention of the grant of the patent: **03.10.2007**
(21) Application number: 99972083.2
(22) Date of filing: 15.11.1999
(51) Int. Cl.: A61B 5/00, A61B 5/1455

(54) **A SENSOR FOR RADIANCE BASED DIAGNOSTICS**
SENSOR FÜR AUF STRAHLUNG BASIERENDER DIAGNOSTIK
CAPTEUR DESTINE A DES DIAGNOSTICS BASES SUR LA RADIANCE

(30) Priority: 16.11.1998 US 108505 P; 28.12.1998 US 113986 P
(43) Date of publication of application: 10.10.2001
(73) Proprietor: S.P.O. Medical Equipment Ltd., 79779 Neve Dkalim (IL)
(72) Inventor: SARUSSI, Israel, 79792 Mobile Mail Hof Aza (IL)
(74) Representative: Vossius & Partner
(86) International application number: PCT/IL1999/000613
(87) International publication number: WO 2000/028888

(56) References cited:
- EP-A- 0 435 500
- EP-A- 0 509 310
- WO-A-99/25241
- US-A- 4 830 014
- US-A- 5 054 488
- US-A- 5 427 093
- US-A- 5 779 631

## Description

### FIELD OF THE INVENTION

The present invention relates to a sensor and system for radiance based diagnostics of body tissues and to a method for radiance based diagnostics of body tissues.

### BACKGROUND OF THE INVENTION

Radiance based diagnostics of body tissues involves radiating a body tissue and obtaining data relating to the transmittance or reflection of the radiated light from the tissue, for analysis of tissue constituents. For example, electro-optical measurement of blood characteristics has been found to be useful in many areas of blood constituent diagnostics, such as glucose levels, oxygen saturation, hematocrit, bilirubin and others. Pulse oximetry is a method for measuring oxygen saturation in the blood, in which two or more wavelengths are radiated through an organ at a point where blood perfuses the organ. Reflective pulse oximetry employs at least one light source and a least one detector which are placed at the same side of an organ. The light source is for radiating the organ and the detector is for receiving the light reflected from the organ. The reflected light is analyzed for measuring the percent of saturated oxygen in the blood.

These methods of body tissue diagnostics usually employ sensors, which are placed on body tissues, and which comprise at least one radiance source for radiating the tissue and at least one radiance detector, for detecting the rays transmitted through or reflected from the tissue. The accuracy of the results obtained in these methods depends, to a great extent, on ensuring that the detector, or detectors, are exposed only to rays which have passed through the examined tissue and not to other rays, such as rays coming directly form the radiance source.

When a sensor is placed on body tissues, especially on stretched tissues (such as over a bone), or due to irregular tissue surface (such as in wrinkles), there might be a small space between the face of the sensor and the tissue, through which some of the rays can pass directly from the radiance source to the detector, thereby adversely affecting the measurement.

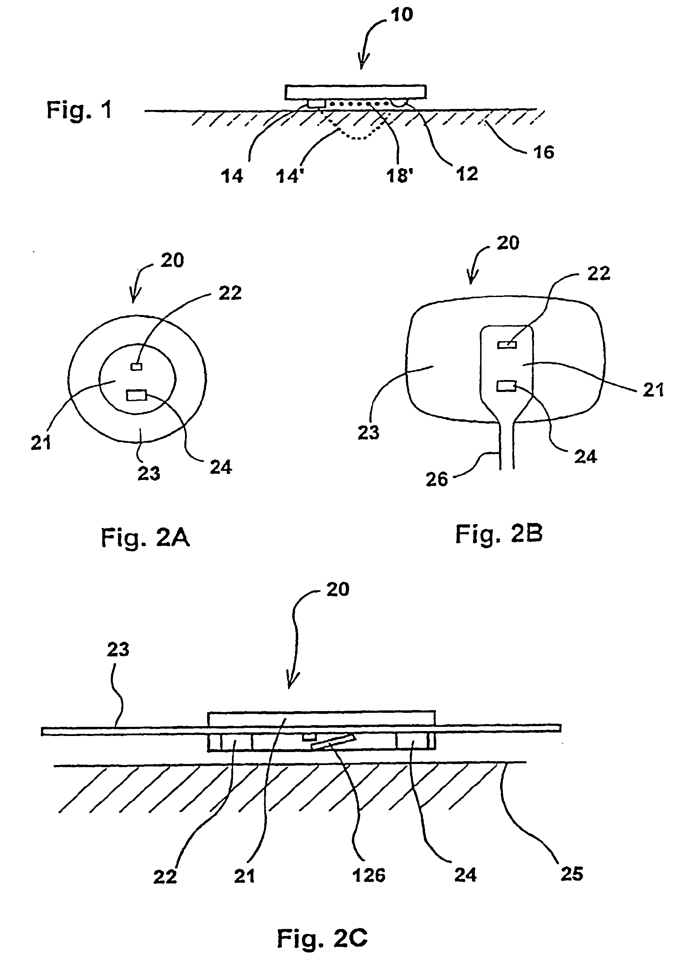

Reference is now made to Fig. 1 which is a schematic side view illustration of a prior art sensor. The sensor, generally referenced 10, contains a light source 12 and a light detector 14. The sensor is placed onto examined tissue 16 and, when operated, light is radiated from light source 12 onto tissue 16. Depending on how the light source 12 is directed, most of the light will pass through the tissue 16 and be partly reflected from the tissue. The reflected light 14' will be received by detector 14 to be analyzed. However, a small fraction of the light 18' radiated from the light source 12 will not pass through the tissue 16 but pass directly to the detector 14, through the small space 18 between the sensor 10 and the tissue 16. Since the detector can not differentiate between the two lights, 14' and 18', the analysis results will be inaccurate.

In US-A-4 830 014, a sensor for trans-illumination of a blood perfused portion of flesh to measure light extinction during trans-illumination is disclosed.

US-A-5 779 631 describes a spectrophotometer system for measuring the metabolic condition of a subject, and in particular an oximeter for determining the oxygenation state of localized body tissue constructed to be worn over a period of activity by a user and comprising a flexible support member which supports, over the localized tissue of interest, at least a pair of spaced apart light sources, and intermediate thereof, at least a pair of wavelength-specific light detectors. Light barrier members serve to reduce light interference directly between the light sources and the detectors and eliminate environmental light interference.

### SUMMARY OF THE INVENTION

It is an object of the present invention to provide a sensor for radiance based diagnostics of body tissues, which ensures that only light which has passed through an examined tissue is received by the sensor's detector. This object is achieved with the features of the claims.

The sensor, which is placed on an examined tissue, comprises a performing component and an adhering component. The performing component, which consists of at least one radiance source for radiating a tissue and at least one detector for detecting the rays transmitted through or reflected from the tissue, protrudes from the plane of the adhering component, in the direction of the examined tissue, when being placed on the tissue. The adhering component is capable of fastening the performing component to the examined tissue. The adhering component may be a tape of any suitable adhering material, which forms a frame around the performing component or, the adhering component may be a tape which overlays the performing component, and which, when being fastened to an examined tissue, covers the performing component, fastening it to the underlying tissue.

The design of the sensor, as described above, ensures that, when the adhering component is placed in contact with the tissue, the performing component presses into the tissue in such a way that the radiance source and detector are sealed off from each other by the tissue, thus, when the sensor is operated, only rays from the radiance source, which have passed through the examined tissue, will be received by the detector.

In, one embodiment of the invention, especially useful for reflective oximetry, the performing component further comprises a raised partition in between the radiance source and the detector, or a wall surrounding either the radiance source or the detector, or both, while separating them from each other. The partition or wall assist in sealing off the detector from the radiance source, to further ensure that no light is received by the detector, directly from the radiance source.

The sensor includes a controlling device which senses external conditions and which is capable of responding to the sensed conditions, such as by arresting the sensor operation when the external conditions indicate inaccurate operation of the sensor. The controlling device is a pressure or proximity detector that will enable sensor operation only when it senses the required proximity to the examined tissue or a pressure which indicates that the performing component is sufficiently pressed on to the examined tissue.

It is further an object of the present invention to provide a system for radiance based diagnostics of body tissues. The system comprises a sensor and a microprocessor that is in electronic communication with a component of the sensor.

The sensor comprises a performing component consisting of at least one radiance source for radiating a tissue and at least one detector for detecting the rays transmitted through or reflected from the tissue, and an adhering component that is capable of fastening the performing component to an examined body tissue. The microprocessor is in electronic communication with the performing component of the sensor for controlling the radiance source and the detector, and for performing analysis of the data received by the detector.

The sensor comprises a controlling device that senses external conditions. The controlling device may be in communication with the microprocessor, providing information to the microprocessor which contributes to its operation of controlling the radiance source and detector. Alternatively, the controlling device may be in direct communication with the radiance source and/or detector, for controlling their operation in accordance with the sensed external conditions.

### BRIEF DESCRIPTION OF THE FIGURES

The present invention will be understood and appreciated more fully from the following detailed description taken in conjunction with the drawings in which:
Fig. 1 is a schematic side view of a prior art sensor;
Figs. 2A and 2B are schematic under views of the sensor according to two embodiments of the present invention;
Fig. 2C is a schematic side view of the sensor according to another embodiment of the present invention.
Figs. 3A and 3B are schematic side views of the sensors of Figs. 2A and 2B, respectively, operable according to an embodiment of the present invention;
Figs. 4A and 4B are schematic under views of the sensors of Figs 2A and 2B, respectively, according to another embodiment of the present invention; and
Figs. 5A and 5B are block diagram illustrations of the operation of a system including the sensors of Figs. 2A and 2B and the sensor of Fig 2C, correspondingly.

### DETAILED DESCRIPTION OF THE INVENTION

The present invention relates to a sensor for radiance based diagnostics of body tissues, which ensures that only light from a radiance source, which has passed through an examined tissue, is received by the sensor's detector, thereby ensuring higher accuracy of results.

The invention will be described in reference to reflective pulse oximetry, but it will be appreciated by persons skilled in the art, that the invention relates to any radiance based method of diagnosing body tissues, reflective or other. The radiance source and detector, may, therefore, be situated on the same side of the examined tissue or on opposing sides, according to the diagnostic method used. The terms "radiance source" and "detector", in the present invention, relate to one or more radiance source or detector. Furthermore, the term "radiance source", in the present invention, refers to a radiance source which is not limited to visible light but may radiate in any wavelength which is suitable for the specific method used.

Reference is now made to Figs. 2A, 2B and 2C, which are schematic under views (Fig. 2A and 2B) and side view (Fig. 2C) of a sensor, generally referenced 20. The sensor 20 comprises a performing component 21 and an adhering component 23. The performing component 21 consists of a radiance source 22 for radiating a tissue and detector 24 for detecting the rays reflected from the tissue. The whole performing component 21, or parts of it (such as the radiance source 22 and/or detector 24), protrude from the plane of the adhering component 23.

The adhering component 23 is a tape for fastening the performing component to an examined body tissue, and for keeping it in place. In Figs. 2A and 2C, the adhering component 23 is an adhesive tape forming a frame around the performing component 21, which contacts both the performing component and underlying tissue.. In Fig. 2B the adhering component 23 is a tape, i.e. made of stretchable material, which overlays the performing component, and which, when being fastened to a body tissue, covers the performing component, fastening it to the underlying tissue. The adhering component 23 may be disposable. It will be appreciated that the adhering component may also be formed as part of the performing component such as a band of adhesive material formed on the surface of the performing component meant to contact and adhere to the examined tissue.

The performing component 21 may be powered through a cable 26 (shown only in Fig. 2B). Alternatively, the sensor 20 may be wireless and operated on a battery.

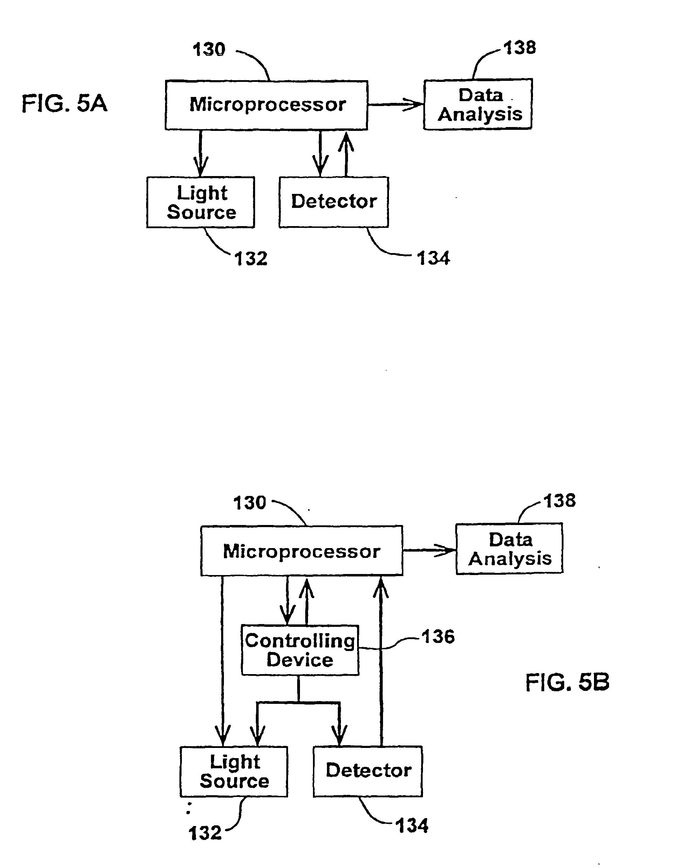

The embodiment described in Fig 2C further comprises a controlling device 126 that may be in communication with either detector 24 or radiance source 22 or both. Controlling device 126 is capable of sensing external conditions and responding to these conditions. A detailed description of controlling device 126 is provided in the description of Fig. 5B.

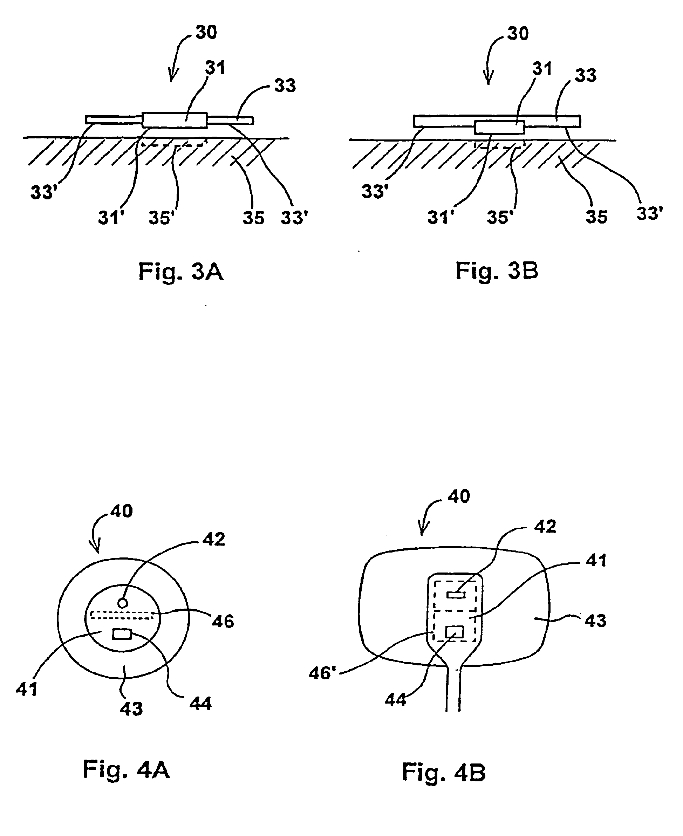

Reference is now made to Figs. 3A and 3B which are schematic side view illustrations of a sensor operable according to the invention. When the sensor, generally referenced 30, is placed on a body tissue 35, such that the adhering component 33 is placed in contact with the tissue 35, the performing component 31, which protrudes from the plane of the adhering component 33, in the direction of the tissue 35, presses into the tissue 35, forming indentation 35'. Adhering component 33 is in contact with performing component 31, such that, the adhering component surface 33' is essentially parallel to the performing component surface 31'. Accordingly, when sensor 30 is fastened to tissue 35, there is essentially no space between the adhering component 33, and the tissue 35. This ensures that the performing component 31 is pressed onto tissue 35 such that light cannot reach the detector either from the outside or directly from the radiance source.

The pressure exerted by performing component 31 on the tissue, fastens the radiance source and detector (not shown) to the tissue so that rays emitted from the radiance source, are unable to reach the detector through any other medium, but the tissue.

The performing component surface 31' can be coated or overlaid with a soft layer, such as a silicone or sponge layer. The soft layer, which does not overlay the radiance source or detector, will contribute to sealing off of the radiance source from the detector when the sensor is pressed onto the tissue 35.

Reference is now made to Figs 4A and 4B which are schematic under view illustrations of a sensor, generally referenced 40, according to another embodiment of the invention. In this embodiment, which is especially suitable for use in reflective oximetry, the performing component 41 of the sensor 40 comprises a raised partition 46 in between the radiance source 42 and the detector 44. The sensor 40 may also comprise a wall 46' surrounding either the radiance source 42 or the detector 44, or both, while separating them from each other (shown in Fig. 4B). When sensor 40 is fastened to a body tissue (as described in Figs. 3A and 3B) by adhering component 43, partition 46 or wall 46', which are raised above the plane of the sensor 40, exert pressure on the tissue and thus seal off the detector 44 from the radiance source 42.

Reference is now made to Fig 5A, which is a block diagram illustration of the operation of a system for radiance based diagnostics of body tissues, which includes the sensors 20 of Figs. 2A and 2B. The system comprises an electronic circuit i.e, microprocessor 130 that is in electronic communication with the performing component of sensor 20 which includes radiance source 132 and detector 134. Microprocessor 130 controls radiance source 132 and/or detector 134 operation. Detector 134 provides microprocessor 130 with data relating to the light detected by it. Microprocessor 130 performs data analysis 138 which may include saving the data and/or further processing it and, optionally, displaying the processed data. Data analysis 138 may be performed in the same physical unit of microprocessor 130 or in a separate unit which is in communication with microprocessor 130. The processed data may further effect microprocessor 130 operation. Thus the microprocessor 130 operation may be fine tuned in accordance with the conditions prevailing in the examined tissue, as they are interperated by the data analysis function of the microprocessor.

It will be appreciated that the term "microprocessor" relates to an electronic circuit capable of communicating with and/or controlling sensor components as described.

Fig 5B is a block diagram illustration of a system for radiance based diagnostics of body tissues, that includes the sensor 20 of Fig. 2C. This sensor includes controlling device 136 that may be in communication with microprocessor 130, on one hand and with radiance source 132 and detector 134, on the other hand, and through which microprocessor 130 may control radiance source 132 and detector 134 operation.

Controlling device 136 responds to external conditions, such as external pressure or external temperature, and may inform microprocessor 130 of these conditions. Microprocessor 130 may be programmed to differentially operate according to different external conditions. For example, controlling device 136 may function to alert microprocessor 130 of the fact that there is insufficient pressure or proximity to the examined tissue, and according to the programmed parameters, microprocessor 130 may either stop sensor 20 operation, or alert an operator by displaying this fact, through data analysis 138.

Alternatively, controlling device 136 may be a switch which responds to pressure or proximity to the examined tissue 35 (see Figs. 3A and 3B). Thus, when the performing component 21, or parts of it, are sufficiently pressed to examined tissue 35, so as to ensure that only light from radiance source 132 that has passed through the examined tissue 35, will be detected by detector 134, the controlling device 136 will be in the ON indication, either enabeling microprocessor 130 operation or enabling communication between the microprocessor. 130 and radiance source 132 and detector. When insufficient pressure or proximity, between the performing component and the examined tissue, is detected by controlling device 136, it will be in the OFF indication, thereby either arresting microprocessor 130 operation or disconnecting the communication between the microprocessor 130 and radiance source 132 and detector 134. Thus the controlling device 136 contributes to the sensor 20 efficiency.

The controlling device 136 may also respond to other external parameters, such as the examined tissue temperature. Thus, the controlling device 136 will either alert an operator, disconnect the microprocessor or disconnect communication between the microprocessor and the performing component, if is senses a temperature different from that of a preprogrammed temperature, such as the body temperature. The sensor 20, which may need to be placed on the examined tissue 35 for a long period, such as over night, may heedlessly fall off the examined tissue 35 and be pressed on to the patient's bed, instead of onto his body. The microprocessor 130 will be alterted to this fact by the controlling device 136, and act accordingly, as described above.

The controlling device 136 may additionally function to ensure that the microprocessor 130 is operative only with the appropriate sensor 20. For example, sensor 20 can include a controlling device 136 that is a pressure detector that is in the OFF indication for the time it takes to achieve appropriate pressure between the sensor and the examined tissue. Accordingly, microprocessor 130 can be programmed to operate only after being shut off for a predetermined period (which would be, for example, the time it takes to achieve appropriate pressure between the sensor and examined tissue). Thus, a sensor 20 that does not include a controlling device 136 will not be operative with the programmed microprocessor 130.

Both in Fig 5A and in Fig 5B, the microprocessor 130 control of the sensor 20 components (radiance source 132 and/or detector 134) may be achieved through connecting wires or by remote control, such as by utilizing radiant energy. Thus the microprocessor 130 may be in close contact with the sensor 20, situated close to the patient's body, or the microprocessor 130 may be at some distance from the sensor 20.

It will be appreciated by persons skilled in the art that the present invention is not limited by what has been particularly shown and described herein above. Rather the scope of the invention is defined by the claims which follow:

## Claims

1. A sensor, for radiance based diagnostics, comprising a performing component (21, 31, 41) and an adhering component (23, 33, 43);
said performing component (21, 31, 41) comprising:
a lower (31') and an upper surface;
at least one radiance source (22, 32, 42) for radiating a tissue; and
at least one detector (24, 44) for detecting rays reflected from said tissue;
said adhering component (23, 33, 43) comprising a lower surface (33') having a greater surface area than the surface area of the performing component (21, 31, 41), said lower surface (33') configured to surround and adhesively adhere to the upper surface of the performing component (21, 31, 41), the lower surface (31') of said performing component (21, 31, 41) extending beyond the lower surface (33') of the adhering component (23, 33, 43);
said performing component (21, 31, 41) comprising means to seal off the detector (24, 44) from the radiance source (22, 32, 42) so that rays emitted from the radiance source (22, 32, 42) are unable to reach the detector (24, 44) through any other medium except from the tissue; and
wherein the lower surface (33') of the adhering component (23, 33, 43) is adhesively attachable to the tissue to form an indentation (35') in the tissue, the surface area of said indentation (35') corresponding to the lower surface (31') of the performing component (21, 31, 41), said indentation (35') preventing external light from being detected by said at least one detector (24, 44);
**characterized by**
a controlling device (126) capable of sensing and responding to external conditions, said controlling device (126, 136) being a pressure or proximity detector configured to enable sensor operation only when the performing component (21, 31, 41) is fastened to the tissue to the extent that the detector (24, 44) only receives rays which are reflected from the tissue.

2. A sensor according to claim 1 wherein the adhering component (23, 33, 43) is a tape of adhering material framing the performing component (21, 31, 41) and which, when fastening the performing component (21, 31, 41) to the tissue, contacts the tissue.

3. A sensor according to claim 1 wherein the adhering component (23, 33, 43) is a tape which, when fastening the performing component (21, 31, 41) to the tissue, overlays the performing component (21, 31, 41) and contacts the tissue.

4. A sensor according to claim 1 wherein the adhering component (23, 33, 43) is formed as part of the performing component (21, 31, 41) and contacts both the performing component (21, 31, 41) and the tissue.

5. A sensor according to claim 1 wherein the performing component (41) further comprises a partition (46) in between the radiance source (42) and the detector (44).

6. A sensor according to claim 5 wherein the partition (46) further surrounds either radiance source (42) or detector (44) or both.

7. A system for radiance based diagnostics comprising a sensor and an electronic circuit (130) in communication with the sensor components and capable of controlling the sensor components operation, said sensor comprising a performing component (21, 31, 41) and an adhering component (23, 33, 43),
said performing component (21, 31, 41) comprising:
a lower (31') and an upper surface;
at least one radiance source (22, 32, 42) for radiating a tissue; and
at least one detector (24, 44) for detecting rays reflected from said tissue;
said adhering component (23, 33, 43) comprising a lower surface (33') having a greater surface area than the surface area of the performing component (21, 31, 41), said lower surface (33') configured to surround and adhesively adhere to the upper surface of the performing component (21, 31, 41), the lower surface (31') of said performing component (21, 31, 41) extending beyond the lower surface (33') of the adhering component (23, 33, 43);
said performing component (21, 31, 41) comprising means to seal off the detector (24, 44) from the radiance source (22, 32, 42) so that rays emitted from the radiance source (22, 32, 42) are unable to reach the detector (24, 44) through any other medium except from the tissue; and
wherein the lower surface (33') of the adhering component (23, 33, 43) is adhesively attachable to the tissue to form an indentation (35') in the tissue, the surface area of said indentation (35') corresponding to the lower surface (31') of the performing component (21, 31, 41), said indentation (35') preventing external light from being detected by said at least one detector (24, 44);
**characterized by**
a controlling device (126) capable of sensing and responding to external conditions, said controlling device (126, 136) being a pressure or proximity detector configured to enable sensor operation only when the performing component (21, 31, 41) is fastened to the tissue to the extent that the detector (24, 44) only receives rays which are reflected from the tissue.

8. A system according to claim 7 wherein the electronic circuit (130) is in communication with and is capable of controlling the operation of either radiance source (22, 32, 42) or detector (24) or both.

9. A system according to claim 7 wherein the adhering component (23, 33, 43) is a tape of adhering material framing the performing component (21, 31, 41) and which, when fastening the performing component (21, 31, 41) to the tissue, contacts the tissue.

10. A system according to claim 7 wherein the adhering component (23, 33, 43) is a tape which, when fastening the performing component (21, 31, 41) to the tissue, overlays the performing component (21, 31, 41) and contacts the tissue.

11. A system according to claim 7 wherein the performing component (41) further comprises a partition (46) in between the radiance source (42) and the detector (44).

12. A system according to claim 11 wherein the partition (46) further surrounds either radiance source (42) or detector (44) or both.

13. A system according to claim 7 wherein the sensor further comprises a controlling device (126) capable of sensing and responding to external conditions and which controlling device (126) is capable of being in communication with sensor components, electronic circuit (130) or both.

14. A system according to claim 13 wherein the electronic circuit (130) controls the sensor component's operation.

15. A system according to claim 13 wherein the electronic circuit (130) is programmed to operate in accordance with specific conditions communicated by the controlling device (126).

## Patentansprüche

1. Sensor für auf Strahlung basierender Diagnostik, der eine ausführende Komponente (21, 31, 41) und eine haftende Komponente (23, 33, 43) aufweist;
wobei die ausführende Komponente (21, 31, 41) aufweist:
eine Unter- (31') und eine Oberseite;
mindestens eine Strahlungsquelle (22, 32, 42) zum Bestrahlen eines Gewebes; und
mindestens einen Detektor (24, 44) zur Detektion von Strahlen, die vom Gewebe reflektiert werden;
wobei die haftende Komponente (23, 33, 43) eine Unterseite (33') mit einer größeren Flächenausdehnung als die Flächenausdehnung der ausführenden Komponente (21, 31, 41) aufweist, wobei die Unterseite (33') so gestaltet ist, daß sie die Oberseite der ausführenden Komponente (21, 31, 41) umgibt und klebend an ihr haftet, wobei sich die Unterseite (31') der ausführenden Komponente (21, 31, 41) über die Unterseite (33') der haftenden Komponente (23, 33, 43) hinaus erstreckt;
wobei die ausführende Komponente (21, 31, 41) eine Einrichtung aufweist, um den Detektor (24, 44) von der Strahlungsquelle (22, 32, 42) abzuschotten, so daß Strahlen, die aus der Strahlungsquelle (22, 32, 42) emittiert werden, den Detektor (24, 44) durch kein anderes Medium außer von dem Gewebe erreichen können; und
wobei die Unterseite (33') der haftenden Komponente (23, 33, 43) klebend an das Gewebe anbringbar ist, um eine Einbuchtung (35') im Gewebe zu bilden, wobei die Flächenausdehnung der Einbuchtung (35') der Unterseite (31') der ausführenden Komponente (21, 31, 41) entspricht, wobei die Einbuchtung (35') verhindert, daß Fremdlicht durch den mindestens einen Detektor (24, 44) detektiert wird; **gekennzeichnet durch**
eine Steuervorrichtung (126), die imstande ist, äußere Bedingungen zu erfassen und auf sie zu reagieren, wobei die Steuervorrichtung (126, 136) ein Druck- oder Näherungsdetektor ist, der so gestaltet ist, daß ein Sensorbetrieb nur ermöglicht wird, wenn die ausführende Komponente (21, 31, 41) derart am Gewebe befestigt ist, daß der Detektor (24, 44) nur Strahlen empfängt, die von dem Gewebe reflektiert werden.

2. Sensor nach Anspruch 1, wobei die haftende Komponente (23, 33, 43) ein Band eines haftenden Materials ist, das die ausführende Komponente (21, 31, 41) umrahmt und das, wenn die ausführende Komponente (21, 31, 41) am Gewebe befestigt ist, das Gewebe berührt.

3. Sensor nach Anspruch 1, wobei die haftende Komponente (23, 33, 43) ein Band ist, das, wenn die ausführende Komponente (21, 31, 41) am Gewebe befestigt ist, die ausführende Komponente (21, 31, 41) überzieht und das Gewebe berührt.

4. Sensor nach Anspruch 1, wobei die haftende Komponente (23, 33, 43) als Teil der ausführenden Komponente (21, 31, 41) ausgebildet ist und sowohl die ausführende Komponente (21, 31, 41) als auch das Gewebe berührt.

5. Sensor nach Anspruch 1, wobei die ausführende Komponente (41) ferner eine Abtrennung (46) zwischen der Strahlungsquelle (42) und dem Detektor (44) aufweist.

6. Sensor nach Anspruch 5, wobei die Abtrennung (46) ferner entweder die Strahlungsquelle (42) oder den Detektor (44) oder beide umgibt.

7. System für auf Strahlung basierende Diagnostik, das einen Sensor und eine elektronische Schaltung (130) aufweist, die mit den Sensorkomponenten in Verbindung steht und zur Steuerung des Sensorkomponentenbetriebs imstande ist, wobei der Sensor eine ausführende Komponente (21, 31, 41) und eine haftende Komponente (23, 33, 43) aufweist,
wobei die ausführende Komponente (21, 31, 41) aufweist:
eine Unter- (31') und eine Oberseite;
mindestens eine Strahlungsquelle (22, 32, 42) zum Bestrahlen eines Gewebes; und
mindestens einen Detektor (24, 44) zur Detektion von Strahlen, die vom Gewebe reflektiert werden;
wobei die haftende Komponente (23, 33, 43) eine Unterseite (33') mit einer größeren Flächenausdehnung als die Flächenausdehnung der ausführenden Komponente (21, 31, 41) aufweist, wobei die Unterseite (33') so gestaltet ist, daß sie die Oberseite der ausführenden Komponente (21, 31, 41) umgibt und klebend an ihr haftet, wobei sich die Unterseite (31') der ausführenden Komponente (21, 31, 41) über die Unterseite (33') der haftenden Komponente (23, 33, 43) hinaus erstreckt;
wobei die ausführende Komponente (21, 31, 41) eine Einrichtung aufweist, um den Detektor (24, 44) von der Strahlungsquelle (22, 32, 42) abzuschotten, so daß Strahlen, die aus der Strahlungsquelle (22, 32, 42) emittiert werden, den Detektor (24, 44) durch kein anderes Medium außer aus dem Gewebe erreichen können; und
wobei die Unterseite (33') der haftenden Komponente (23, 33, 43) klebend an das Gewebe anbringbar ist, um eine Einbuchtung (35') im Gewebe zu bilden, wobei die Flächenausdehnung der Einbuchtung (35') der Unterseite (31') der ausführenden Komponente (21, 31, 41) entspricht, wobei die Einbuchtung (35') verhindert, daß Fremdlicht durch den mindestens einen Detektor (24, 44) detektiert wird; **gekennzeichnet durch**
eine Steuervorrichtung (126), die imstande ist, äußere Bedingungen zu erfassen und auf sie zu reagieren, wobei die Steuervorrichtung (126, 136) ein Druck- oder Näherungsdetektor ist, der so gestaltet ist, daß ein Sensorbetrieb nur ermöglicht wird, wenn die ausführende Komponente (21, 31, 41) derart am Gewebe befestigt ist, daß der Detektor (24, 44) nur Strahlen empfängt, die von dem Gewebe reflektiert werden.

8. System nach Anspruch 7, wobei die elektronische Schaltung (130) entweder mit der Strahlungsquelle (22, 32, 42) oder dem Detektor (24) oder beiden in Verbindung steht und imstande ist, den Betrieb entweder der Strahlungsquelle (22, 32, 42) oder dem Detektor (24) oder beiden zu steuern.

9. System nach Anspruch 7, wobei die haftende Komponente (23, 33, 43) ein Band eines haftenden Materials ist, das die ausführende Komponente (21, 31, 41) umrahmt und das, wenn die ausführende Komponente (21, 31, 41) am Gewebe befestigt ist, das Gewebe berührt.

10. System nach Anspruch 7, wobei die haftende Komponente (23, 33, 43) ein Band ist, das, wenn die ausführende Komponente (21, 31, 41) am Gewebe befestigt ist, die ausführende Komponente (21, 31, 41) überzieht und das Gewebe berührt.

11. System nach Anspruch 7, wobei die ausführende Komponente (41) ferner eine Abtrennung (46) zwischen der Strahlungsquelle (42) und dem Detektor (44) aufweist.

12. System nach Anspruch 11, wobei die Abtrennung (46) ferner entweder die Strahlungsquelle (42) oder den Detektor (44) oder beide umgibt.

13. System nach Anspruch 7, wobei der Sensor ferner eine Steuervorrichtung (126) aufweist, die imstande ist, äußere Bedingungen zu erfassen und auf sie zu reagieren, und wobei die Steuervorrichtung (126) mit Sensorkomponenten, der elektronischen Schaltung (130) oder beiden in Verbindung stehen kann.

14. System nach Anspruch 13, wobei die elektronische Schaltung (130) den Betrieb der Sensorkomponente steuert.

15. System nach Anspruch 13, wobei die elektronische Schaltung (130) so programmiert ist, daß sie gemäß spezifischer Bedingungen arbeitet, die durch die Steuervorrichtung (126) übermittelt werden.

## Revendications

1. Capteur destiné à des diagnostics basés sur la radiance, comprenant un composant d'exécution (21, 31, 41) et un composant adhésif (23, 33, 43) ;
ledit composant d'exécution (21, 31, 41) comprenant :
une surface inférieure (31') et une surface supérieure ;
au moins une source de radiance (22, 32, 42) pour irradier un tissu ; et
au moins un détecteur (24, 44) pour détecter des rayons réfléchis par ledit tissu;
ledit composant adhésif (23, 33, 43) comprenant une surface inférieure (33') ayant une zone de surface supérieure à la zone de surface du composant d'exécution (21, 31, 41), ladite surface inférieure (33') étant configurée pour entourer et adhérer de manière adhésive à la surface supérieure du composant d'exécution (21, 31, 41), la surface inférieure (31') dudit composant d'exécution (21, 31, 41) s'étendant au-delà de la surface inférieure (33') du composant adhésif (23, 33, 43);
ledit composant d'exécution (21, 31, 41) comprenant des moyens pour assurer l'étanchéité du détecteur (24, 44) par rapport à la source de radiance (22, 32, 42) de sorte que les rayons émis depuis la source de radiance (22, 32, 42) ne puissent pas atteindre le détecteur (24, 44) à travers tout autre milieu que le tissu ; et
dans lequel la surface inférieure (33') du composant adhésif (23, 33, 43) peut être fixée de manière adhésive au tissu pour former une indentation (35') dans le tissu, la zone de surface de ladite indentation (35') correspondant à la surface inférieure (31') du composant d'exécution (21, 31, 41), ladite indentation (35') empêchant la lumière extérieure d'être détectée par ledit au moins un détecteur (24, 44) ;
**caractérisé par**
un dispositif de commande (126) capable de détecter et de répondre à des conditions externes, ledit dispositif de commande (126, 136) étant un détecteur de pression ou de proximité configuré pour permettre le fonctionnement du capteur uniquement lorsque le composant d'exécution (21, 31, 41) est fixé au tissu de sorte que le détecteur (24, 44) ne reçoive que les rayons qui sont réfléchis par le tissu.

2. Capteur selon la revendication 1, dans lequel le composant adhésif (23, 33, 43) est une bande de matériau adhésif encadrant le composant d'exécution (21, 31, 41) et qui, lors de la fixation du composant d'exécution (21, 31, 41) au tissu, vient en contact avec le tissu.

3. Capteur selon la revendication 1, dans lequel le composant adhésif (23, 33, 43) est une bande qui, lors de la fixation du composant d'exécution (21, 31, 41) au tissu, recouvre le composant d'exécution (21, 31, 41) et vient en contact avec le tissu.

4. Capteur selon la revendication 1, dans lequel le composant adhésif (23, 33, 43) est formé comme faisant partie du composant d'exécution (21, 31, 41) et vient en contact avec le composant d'exécution (21, 31, 41) et le tissu.

5. Capteur selon la revendication 1, dans lequel le composant d'exécution (41) comprend en outre une séparation (46) entre la source de radiance (42) et le détecteur (44).

6. Capteur selon la revendication 5, dans lequel la séparation (46) entoure en outre la source de radiance (42) ou le détecteur (44) ou les deux.

7. Système destiné à des diagnostics basés sur la radiance comprenant un capteur et un circuit électronique (130) en communication avec les composants de capteur et capable de commander le fonctionnement des composants de capteur, ledit capteur comprenant un composant d'exécution (21, 31, 41) et un composant adhésif (23, 33, 43) ;
ledit composant d'exécution (21, 31, 41) comprenant :
une surface inférieure (31') et une surface supérieure;
au moins une source de radiance (22, 32, 42) pour irradier un tissu ; et
au moins un détecteur (24, 44) pour détecter des rayons réfléchis par ledit tissu ;
ledit composant adhésif (23, 33, 43) comprenant une surface inférieure (33') ayant une zone de surface supérieure à la zone de surface du composant d'exécution (21, 31, 41), ladite surface inférieure (33') étant configurée pour entourer et adhérer de manière adhésive à la surface supérieure du composant d'exécution (21, 31, 41), la surface inférieure (31') dudit composant d'exécution (21, 31, 41) s'étendant au-delà de la surface inférieure (33') du composant adhésif (23, 33, 43) ;
ledit composant d'exécution (21, 31, 41) comprenant des moyens pour assurer l'étanchéité du détecteur (24, 44) par rapport à la source de radiance (22, 32, 42) de sorte que les rayons émis depuis la source de radiance (22, 32, 42) ne puissent pas atteindre le détecteur (24, 44) à travers tout autre milieu que le tissu ; et
dans lequel la surface inférieure (33') du composant adhésif (23, 33, 43) peut être fixée de manière adhésive au tissu pour former une indentation (35') dans le tissu, la zone de surface de ladite indentation (35') correspondant à la surface inférieure (31') du composant d'exécution (21, 31, 41), ladite indentation (35') empêchant la lumière extérieure d'être détectée par ledit au moins un détecteur (24, 44) ;
**caractérisé par**
un dispositif de commande (126) capable de détecter et de répondre à des conditions externes, ledit dispositif de commande (126, 136) étant un détecteur de pression ou de proximité configuré pour permettre le fonctionnement du capteur uniquement lorsque le composant d'exécution (21, 31, 41) est fixé au tissu de sorte que le détecteur (24, 44) ne reçoive que les rayons qui sont réfléchis par le tissu.

8. Système selon la revendication 7, dans lequel le circuit électronique (130) est en communication avec et est capable de commander le fonctionnement de la source de radiance (22, 32, 42) ou du détecteur (24) ou des deux.

9. Système selon la revendication 7, dans lequel le composant adhésif (23, 33, 43) est une bande de matériau adhésif encadrant le composant d'exécution (21, 31, 41) et qui, lors de la fixation du composant d'exécution (21, 31, 41) au tissu, vient en contact avec le tissu.

10. Système selon la revendication 7, dans lequel le composant adhésif (23, 33, 43) est une bande qui, lors de la fixation du composant d'exécution (21, 31, 41) au tissu, recouvre le composant d'exécution (21, 31, 41) et vient en contact avec le tissu.

11. Système selon la revendication 7, dans lequel le composant d'exécution (41) comprend en outre une séparation (46) entre la source de radiance (42) et le détecteur (44).

12. Système selon la revendication 11, dans lequel la séparation (46) entoure en outre la source de radiance (42) ou le détecteur (44) ou les deux.

13. Système selon la revendication 7, dans lequel le capteur comprend en outre un dispositif de commande (126) capable de détecter et de répondre à des conditions externes et ce dispositif de commande (126) est capable d'être en communication avec les composants de capteur, le circuit électronique (130) ou les deux.

14. Système selon la revendication 13, dans lequel le circuit électronique (130) commande le fonctionnement du composant de capteur.

15. Système selon la revendication 13, dans lequel le circuit électronique (130) est programmé pour fonctionner selon des conditions spécifiques communiquées par le dispositif de commande (126).
